(19) Europäisches Patentamt
European Patent Office
Office européen des brevets

(11) Veröffentlichungsnummer: **0 163 996**
**A1**

(12) **EUROPÄISCHE PATENTANMELDUNG**

(21) Anmeldenummer: **85105967.5**

(22) Anmeldetag: **15.05.85**

(51) Int. Cl.⁴: **C 07 C 69/757**, C 07 C 109/12, C 07 C 147/06

(30) Priorität: **26.05.84 DE 3419750**

(43) Veröffentlichungstag der Anmeldung: **11.12.85**
**Patentblatt 85/50**

(84) Benannte Vertragsstaaten: **BE CH DE FR GB IT LI NL**

(71) Anmelder: **BAYER AG, Konzernverwaltung RP**
**Patentabteilung, D-5090 Leverkusen 1 Bayerwerk (DE)**

(72) Erfinder: **Hanack, Michael, Prof. Dr.,**
**Panoramastrasse 39, D-7400 Tübingen 7 (DE)**
Erfinder: **Wunde, Christian, Dr., Taubenstrasse 18,**
**D-7407 Rottenburg 5 (DE)**

(54) **2,2-Dimethyl-3-formyl-cyclopropane und Verfahren zur ihrer Herstellung.**

(57) Die vorliegende Erfindung betrifft neue 2,2-Dimethyl-3-formyl-cyclopropane der Formel

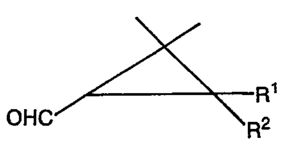

I

in welcher
$R^1$ und $R^2$ für CN, $COC_{1-4}$-Alkyl, CO-Halogen, $COOC_{1-4}$-Alkyl stehen, Verfahren zu ihrer Herstellung, neue Zwischenprodukte zur Durchführung dieses Verfahrens sowie Verfahren zur Herstellung dieser Zwischenprodukte.

Die Cyclopropane der Formel I können z.B. mit an sich bekannten Methoden in Permethrinsäurederivate übergeführt werden, welche als Ausgangsstoffe für Pyrethroide eingesetzt werden.

BAYER AKTIENGESELLSCHAFT 5090 Leverkusen, Bayerwerk
Konzernverwaltung RP
Patentabteilung

2,2-Dimethyl-3-formyl-cyclopropane und Verfahren zu ihrer Herstellung

Die vorliegende Erfindung betrifft neue 2,2-Dimethyl-3-formyl-cyclopropane, ein Verfahren zu ihrer Herstellung, neue Zwischenprodukte zur Durchführung dieses Verfahrens sowie Verfahren zur Herstellung dieser Zwischenprodukte.

Es ist bereits bekannt geworden, Cyclopropan-1,1-dicarbonsäureester herzustellen (DE-OS 2 606 635). Dazu wird 1,1-Dimethyl-2-propenylmalonsäurediethylester mit Tetrachlorkohlenstoff zu 1,1-Dimethyl-2-chlor-4,4,4-trichlor-butyl-malonsäurediethylester umgesetzt und dieser in Gegenwart von Basen cyclisiert. Diese Synthese hat den Nachteil, daß sie von kostspieligen Ausgangsmaterialien ausgeht.

Außerdem sind nach dieser Synthese nur vinylsubstituierte Cyclopropancarbonsäureester zugänglich. Um die als wichtige Zwischenprodukte dienenden Formylcyclopropane herzustellen, muß der Vinylrest ozonolytisch gespalten werden. Das Verfahren zur Herstellung von Formylcyclopropanderivaten wird damit wirtschaftlich ohne Interesse.

1. Es wurden die neuen 2,2-Dimethyl-3-formyl-cyclopropane der Formel I

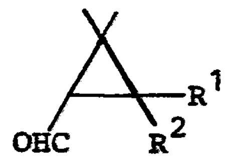

I

in welcher

$R^1$ und $R^2$ für CN, $COC_{1-4}$-Alkyl, CO-Halogen, $COOC_{1-4}$-Alkyl stehen,

gefunden.

2. Es wurde gefunden, daß die neuen 2,2-Dimethyl-3-formyl-cyclopropane der Formel I

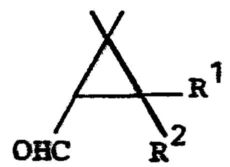

I

in welcher

$R^1$ und $R^2$ für CN, $COC_{1-4}$-Alkyl, CO-Halogen, $COOC_{1-4}$-Alkyl stehen,

erhalten werden, indem man 2,2-Dimethyl-3-formyl-hydrazon-cyclopropane der Formel II

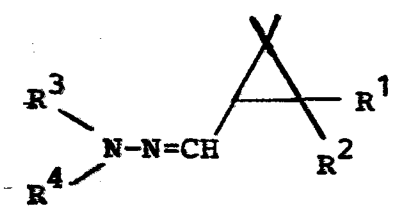

II

in welcher

$R^1$ und $R^2$ die oben angegebene Bedeutung haben und

$R^3$ und $R^4$ für Wasserstoff, $C_{1-4}$-Alkyl oder gegebenenfalls substituiertes Phenyl stehen oder gemeinsam eine Alkylbrücke bilden, die gegebenenfalls durch Heteroatome unterbrochen und gegebenenfalls substituiert ist,

in an sich bekannter Weise spaltet.

3. Es wurden die neuen 2,2-Dimethyl-3-formylhydrazoncyclopropane der Formel II

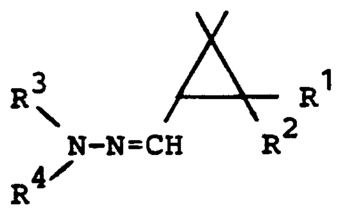

II

in welcher

$R^1$ und $R^2$ die oben angegebene Bedeutung haben und

$R^3$ und $R^4$ für Wasserstoff, $C_{1-4}$-Alkyl oder gegebenenfalls substituiertes Phenyl stehen oder gemeinsam eine Alkylbrücke bilden, die gegebenenfalls durch Heteroatome unterbrochen und gegebenenfalls substituiert ist,

gefunden.

4. Es wurde gefunden, daß die neuen 2,2-Dimethyl-3-formylhydrazon-cyclopropane der Formel II

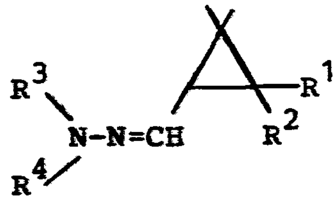

II

in welcher

$R^1$ und $R^2$ die oben angegebene Bedeutung haben und

$R^3$ und $R^4$ für Wasserstoff, $C_{1-4}$-Alkyl oder gegebenenfalls substituiertes Phenyl stehen oder gemeinsam eine Alkylbrücke bilden, die gegebenenfalls durch Heteroatome unterbrochen und gegebenenfalls substituiert ist,

erhalten werden, indem man Hydrazone der Formel III

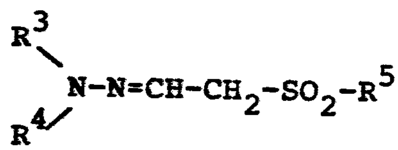

III

in welcher

$R^3$ und $R^4$ die oben angegebene Bedeutung haben und

$R^5$ für gegebenenfalls substituiertes Aryl steht,

mit Alkylenen der Formel IV

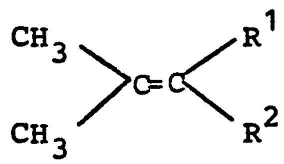

IV

in welcher

$R^1$ und $R^2$ die oben angegebene Bedeutung haben

in Gegenwart von Basen umsetzt.

5. Es wurden die neuen Hydrazone der Formel III

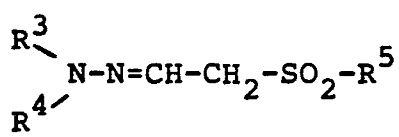

III

in welcher

$R^3$ und $R^4$ die oben angegebene Bedeutung haben und
$R^5$ für gegebenenfalls substituiertes Aryl steht,

gefunden.

6. Es wurde gefunden, daß die neuen Hydrazone der Formel III

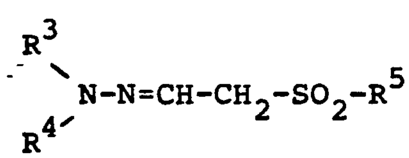

III

in welcher

$R^3$ und $R^4$ die oben angegebene Bedeutung haben und
$R^5$ für gegebenenfalls substituiertes Aryl steht,

erhalten werden, indem man

a) Sulfonylacetaldehydhydrate der Formel V

$$R^5-SO_2-CH_2-CHO.2H_2O \qquad V$$

in welcher

$R^5$ die oben angegebene Bedeutung hat,

mit Hydrazinen der Formel VI

$$H_2N-NR^3R^4 \qquad VI$$

in welcher

$R^3$ und $R^4$ die oben angegebene Bedeutung haben,

umsetzt oder

b) Sulfonylacetaldehydacetale der Formel VII

$$R^5-SO_2-CH_2-CH(OR^6)_2 \qquad VII$$

in welcher

$R^5$ die oben angegebene Bedeutung hat und
$R^6$ für $C_{1-4}$-Alkyl, insbesondere Methyl oder Ethyl oder beide Reste $R^6$ für eine $C_{2-3}$-Alkylenbrücke stehen,

mit Hydrazinen der Formel VI (oben) in Gegenwart saurer Katalysatoren umsetzt oder

c) Sulfonylethanol der Formel VIII

$$R^5SO_2CH_2CH_2-OH \qquad VIII$$

in welcher

$R^5$ die oben angegebene Bedeutung hat,

zunächst in einer ersten Stufe zum Aldehyd oxidiert und anschließend in einer zweiten Stufe den Aldehyd mit Hydrazinen der Formel VI (oben) umsetzt, oder

d) Sulfonylchloride der Formel IX

$$R^5SO_2Cl \qquad IX$$

in welcher

$R^5$ die oben angegebene Bedeutung hat,

mit Acetaldehydhydrazonen der Formel X

$$CH_3-CH=N-NR^3R^4 \qquad X$$

in welcher

$R^3$ und $R^4$ die oben angegebene Bedeutung haben,

in Gegenwart von organischen Lithium-Basen, z.B. Lithiumdiisopropylamid oder Butyl-lithium, umsetzt.

Unter Verwendung der neuen Verbindungen der Formel I ist es z.B. möglich, den wirtschaftlich bedeutsamen Permethrinsäureester der Formel XII gemäß folgender Reaktionssequenz herzustellen:

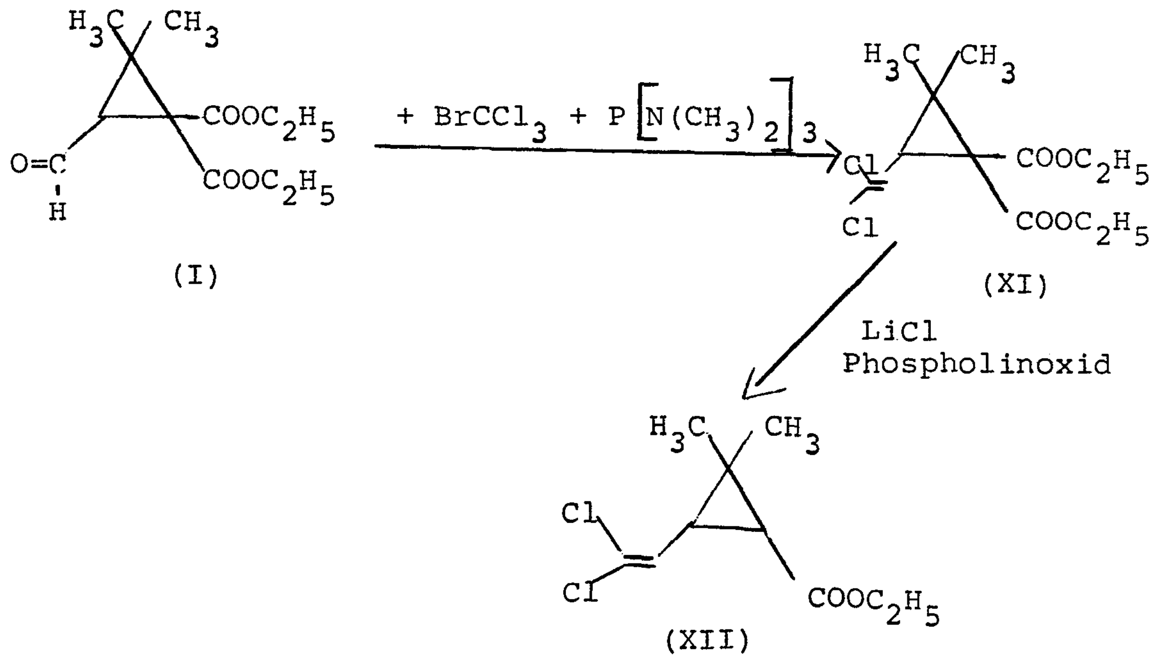


(I) (XI) (XII)

Die Aldehydgruppe wird dabei mit Hilfe einer an sich bekannten Reaktion (W.G. Taylor, Synthesis 554 (1980)) in die aus der DE-OS 27 32 455 und aus der DE-OS 26 06 635 bekannte Verbindung XI (Diester) umgewandelt, die nach an sich bekannten Methoden zum Permethrinsäureester XII durch Decarboxethylierung umgesetzt werden kann. Diese Decarboxethylierung kann z.B. mit Dimethylsulfoxid/$H_2O$/NaCl oder mit Phospholinoxid/$H_2O$/Alkalichlorid erfolgen (Literatur DE-OS 25 36 145 bzw. DE-OS 26 38 453).

Bevorzugt sind Verbindungen der Formel I, in welcher $R^1$ und $R^2$ für gleiche Reste -CN, $-COCH_3$, $-COOCH_3$, $-COOC_2H_5$ stehen.

Das Verfahren 2 zur Herstellung der Verbindungen der Formel I läßt sich durch folgendes Formelschema darstellen:

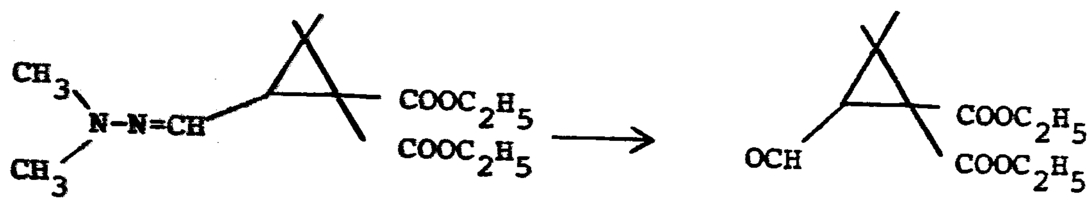

Die Spaltung der Hydrazone der Formel II zu den Verbindungen der Formel I erfolgt in an sich bekannter Weise (Corey et al., Chem. Ber. 111 S. 1337-1383 (1978)).

a.) So läßt sich nach Überführung des Hydrazons II in ein Quartärsalz und anschließende saure wäßrige Hydrolyse der Aldehyd I freisetzen entsprechend der Formel:

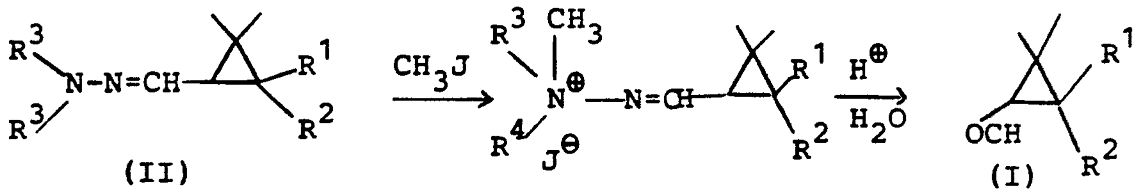


Dabei wird das Hydrazon II mehrere Stunden mit einem Alkylhalogenid wie z.B. Methyljodid unter Rückfluß gekocht, überschüssiges Methyljodid im Vakuum abgezogen und das feste Quartärsalz in verdünnter Salzsäure aufgelöst. Nach Zugabe eines unpolaren organischen Lösungsmittels und Rühren sammelt sich der Aldehyd I in der organischen Phase.

b.) So läßt sich das Hydrazon II oxidativ mit Perjodsäure in den Aldehyd überführen entsprechend der Formel:

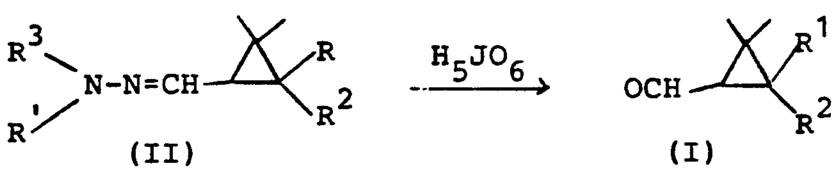

Dabei wird Hydrazon II in einem Lösungsmittel wie z.B. THF/Wasser gelöst und mit einer wäßrigen Lösung einer Persäure wie z.B. Perjodsäure versetzt, welche z.B. mit Natriumacetat/Essigsäure auf pH = 4,5 eingestellt wurde. Mehrstündiges Rühren bei Raumtemperatur setzt Aldehyd I frei.

Bevorzugt werden bei Verfahren 2 Verbindungen der Formel II eingesetzt, in denen $R^1$ und $R^2$ die oben angegebene bevorzugte Bedeutung haben und $R^3$ und $R^4$ für gleiche oder verschiedene Reste der Gruppe Wasserstoff, $C_{1-4}$-Alkyl, gegebenenfalls durch $C_{1-4}$-Alkyl, Halogen wie Fluor, Chlor, Brom, Nitro substituiertes Phenyl steht. Besonders bevorzugt steht $R^3$ für Wasserstoff oder Methyl, $R^4$ für Methyl oder gegebenenfalls durch Nitro substituiertes Phenyl. Im einzelnen seien genannt:

2,2-Dimethyl-3-formyl-dimethylhydrazon-1,1-cyclopropan-dicarbonsäure-methylester, -ethylester,
2,2-Dimethyl-3-formyl-phenylhydrazon-1,1-cyclopropan-dicarbonsäure-methylester, -ethylester,
2,2-Dimethyl-3-formyl-2,4-dinitrophenylhydrazon-1,1-cyclopropan-dinitril, -dicarbonsäuremethylester, -dicarbonsäure-ethylester.

Das Verfahren 4 zur Herstellung der Verbindungen der Formel II läßt sich durch folgendes Formelschema darstellen:

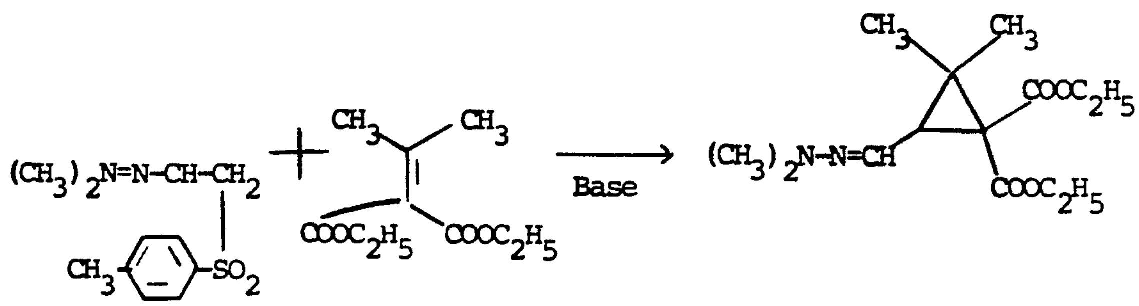


Der Ablauf dieses Verfahrens war überraschend. So konnte nicht erwartet werden, daß das Hydrazon der Formel III in so einfacher Weise und mit guter Ausbeute mit dem Alkylen der Formel IV reagiert.

Bevorzugt werden bei Verfahren 4 Verbindungen der Formel III eingesetzt, in denen $R^3$ und $R^4$ die oben angegebene bevorzugte Bedeutung haben und $R^5$ bevorzugt für Phenyl steht, das gegebenenfalls durch Halogen, Nitro, $C_{1-4}$-Alkyl substituiert ist. Besonders bevorzugt steht $R^5$ für gegebenenfalls methylsubstituiertes Phenyl. Im einzelnen seien folgende Verbindungen der Formel III genannt:

p-Toluolsulfonylacetaldehyd-N,N-dimethylhydrazon,
Phenylsulfonylacetaldehyd-N,N-dimethylhydrazon,
p-Toluolsulfonylacetaldehyd-phenylhydrazon,
Phenylsulfonylacetaldehyd-phenylhydrazon,
p-Toluolsulfonylacetaldehyd-2,4-dinitrophenylhydrazon,
Phenylsulfonylacetaldehyd-2,4-dinitrophenylhydrazon,
p-Toluolsulfonylacetaldehyd-4-nitrophenylhydrazon,
Phenylsulfonylacetaldeyhd-4-nitrophenylhydrazon.

Bevorzugt werden bei Verfahren 4 Verbindungen der Formel IV eingesetzt, in denen $R^1$ und $R^2$ die oben angegebene bevorzugte Bedeutung hat. Im einzelnen seien folgende Verbindungen der Formel IV genannt:

ß,ß-Dimethylacrylsäureethylester,
ß,ß-Dimethylacrylsäuremethylester,
ß,ß-Dimethylacrylonitril,
Isopropylidenmalonsäurediethylester,
Isopropylidenmalonsäuredimethylester,
Isopropylidencyanessigsäureethylester,
Isopropylidencyanessigsäuremethylester,
Isopropylidenmalodinitril,
Isopropylidenacetessigsäureethylester,
Isopropylidenacetessigsäuremethylester.

Verfahren 4 wird in Gegenwart von Basen durchgeführt. Als solche dienen starke anorganische und organische Basen wie Alkali- oder Erdalkali-alkoholate, -hydroxide, -carbonate. Besonders bevorzugt sind Alkalialkoholate wie Natrium- oder Kalium-methylat oder -ethylat, n-Butyllithium oder Lithiumdiisopropylamid. Kupfersalze können zusammen mit Dialkylsulfiden als Katalysatoren eingesetzt werden (Corey et al., Chem. Ber. 111, 1362-1383 (1978)).

Verfahren 4 wird in Gegenwart inerter organischer Verdünnungsmittel durchgeführt. Als solche dienen Kohlenwasserstoff, wie Benzol, Toluol, Ether, THF, DMF, DMSO, HMPT, Petrolether.

Das Hydrazon der Formel III wird unter Verwendung von einem bis vier Äquivalenten Base in sein Carbanion überführt und reagiert mit einem Alkylen der Formel IV, welches in der Menge von einem bis fünf Äquivalenten dem Reaktionsgemisch zugegeben wird, zu dem Hydrazon der Formel II. Die Reaktionstemperatur liegt in dem Bereich von -78°C bis 120°C. Die Reaktionszeit liegt in dem Bereich von 1 Stunde bis 5 Tagen.

Das Verfahren 6 zur Herstellung der Hydrazone der Formel III läßt sich durch folgendes Formelschema darstellen:

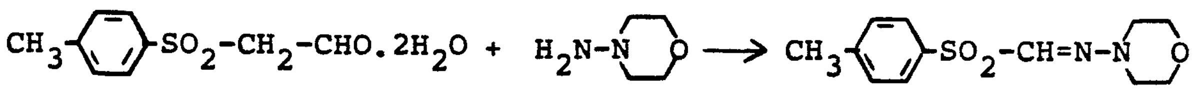

Das Verfahren wird in an sich bekannter Weise durchgeführt (Corey et al., Chem. Ber. 111 (1978) 1337-1361).

Es werden hierbei inerte organische Lösungsmittel verwendet, die mit Wasser Azeotrope bilden können, wie Benzol, Toluol, Tetrachlorkohlenstoff, Chloroform, Methylenchlorid usw. Als Katalysatoren werden anorganische oder organische Säuren verwendet, wie z.B. Schwefelsäure, Salzsäure, p-Toluolsulfonsäure. Aldehyde der Formel V werden zusammen mit Hydrazinen der Formel VI zu Hydrazonen der Formel III umgesetzt, indem Wasser azeotrop aus dem Reaktionsgemisch entfernt wird. Die Reaktionszeiten liegen in dem Bereich von 1 Stunde bis 3 Tagen.

Die Hydrazone der Formel III können jedoch auch nach anderen an sich bekannten Verfahren hergestellt werden. Diese lassen sich durch die folgenden Formelschemata darstellen:

a)

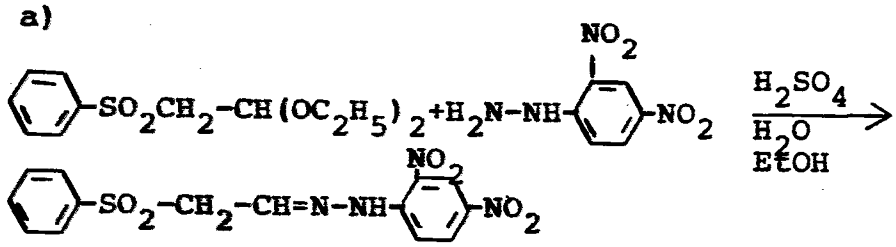


(vgl. Organikum VEB-Verlag Auflage 1976 s. 484)

b)

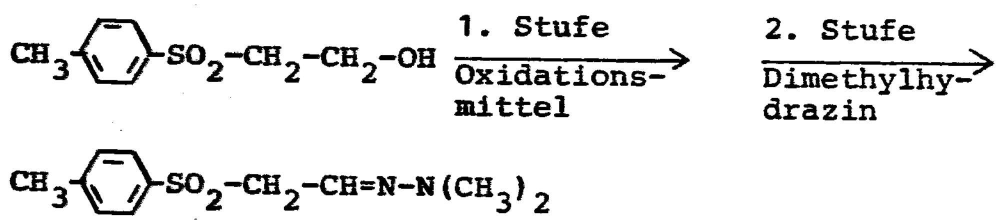


(vgl. Montanari Synthesis /1979) S. 134; Kohn et al. Chem. Ber. (1921) s. 320; Corey et al., Chem. Ber. 111 (1978) S. 1337-1361)

c)

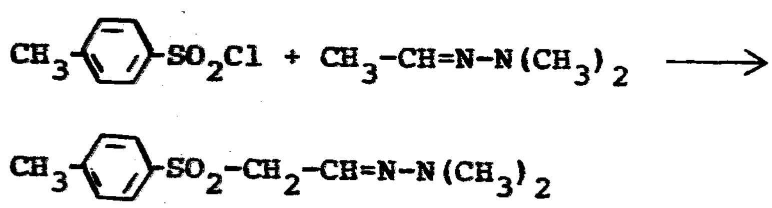

(vgl. Enders et al., Tetrahedron Letters 32 (1978) S. 2853-2856; Corey et al. Chem. Ber. 111 (1978) S. 1337-1383).

Bevorzugte Aldehyde, die den Hydraten der Formel V oder den Acetalen der Formel VII zugrunde liegen sind Phenylsulfonylacetaldehyd, Tolylsulfonylacetaldehyd, p-Chlorphenylsulfonylacetaldehyd.

Bevorzugte Sulfonylethanole der Formel VIII sind Benzolsulfonylethanol, Tolylsulfonylethanol.

Bevorzugte Sulfonylchloride der Formel IX sind Benzolsulfonylchlorid, Tosylchlorid.

Bevorzugte Hydrazine der Formel VI sind Phenylhydrazin, 2,4-Dinitrophenylhydrazin, Monomethyl- und Dimethylhydrazin, N-Aminomorpholin.

Bevorzugte Acetaldehydhydrazone der Formel X sind diejenigen, die auf den bevorzugten Hydrazinen der Formel VI basieren.

Die Verbindungen der Formeln V, VII, VIII, IX, VI, X sind bekannt oder lassen sich analog zu bekannten Verfahren herstellen.

Verbindungen der allgemeinen Formel III werden nach Verfahren 6 dargestellt oder alternativ durch folgende Verfahren:

a) Das Acetal der allgemeinen Formel VII wird mit einem Gemisch aus Hydrazin der allgemeinen Formel VI, Alkohol wie Ethanol, Wasser und Säure, wie z.B. Schwefelsäure oder Essigsäure versetzt und gegebenenfalls mehrere Minuten erhitzt. Acetalspaltung und Hydrazonbildung können auf diese Weise in einer Reaktionsstufe durchgeführt werden.

b.) Alkohole der allgemeinen Formel VIII können mit Oxidationsmittel, wie z.B. Chromaten, in Aldehyde der Formel V überführt werden. Besonders geeignet sind Methoden der Phasentransferkatalyse unter Verwendung von quartären Ammoniumsalzen, Kaliumchromat und Mineralsäuren, z.B. Schwefelsäure. Der so entstandene Aldehyd der Formel V kann, wie in Verfahren 6 beschrieben, in das Hydrazon der allgemeinen Formel III überführt werden.

c.) Hydrazone des Acetaldehyds mit der allgemeinen Formel X werden mit organischen Lithiumbasen, wie z.B. Butyllithium oder Lithiumdiisopropylamid, in getrockneten inerten organischen Lösungsmitteln, z.B. Ether oder THF, in ihre Lithiumsalze überführt und bei Temperaturen in bevorzugten Bereich zwischen -78°C und Raumtemperatur mit Tosylchlorid zu Sulfonen der allgemeinen Formel III umgesetzt.

## Experimenteller Teil

a) Verfahren zur Herstellung der 2,2-Dimethyl-3-formyl-cyclopropane der Formel I

### Beispiel 1

0,284 g (0,001 Mol) 2,2-Dimethyl-3-formyl-dimethylhydrazon-cyclopropan-1,1-dicarbonsäureethylester wurden mit 1,5 ml Methyljodid versetzt und über Nacht am Rückfluß gekocht. Davon wurde Methyljodid im Vakuum abdestilliert. Zu dem Rückstand wurden 40 ml 5 %ige HCl gegeben und bis zur Lösung gerührt. Anschließend wurden 30 ml Ether/PE (30/50) 1:1 hinzugegeben und 1 h stark gerührt bei Raumtemperatur. Die Phasen wurden getrennt und die wäßrige Phase dreimal mit Ether extrahiert. Die Etherextrakte wurden einmal mit $NaHCO_3$-Lösung und dreimal mit $H_2O$ gewaschen. Es wurde über $MgSO_4$ getrocknet, filtriert und eingeengt. Es wurden 0,20 g ≙ 82,6 % 2,2-Dimethyl-3-formyl-cyclopropan-1,1-dicarbonsäureethylester erhalten.

1HNMR: 1,1-1,6 ppm 12 Methylprotonen; 2,55 ppm D. 1 Methinproton; 4,0-4,45 ppm Q. 4 Methylenprotonen; 9,5 ppm 1 Methinproton.

Beispiel 2

0,284 g (0,001 Mol) 2,2-Dimethyl-3-formyl-dimethylhydrazon-cyclopropan-1,1-dicarbonsäureethylester werden mit 15 ml THF und 10 ml $H_2O$ versetzt. Es wird eine Lösung von 0,228 g $H_5JO_6$ (0,001 mol) in 5 ml $H_2O$, welche mit NaOAc/HAc auf pH = 4,5 eingestellt wurde, hinzugegeben und 15 h gerührt. Es werden noch einmal 0,228 g $H_5JO_6$ hinzugegeben und weitere 2 h gerührt. Die Aufarbeitung erfolgt, wie in Beispiel 1 angegeben. Man erhält 0,30 g eines Substanzgemisches, welches 90 % 2,2-Dimethyl-3-formyl-cyclopropan-1,1-dicarbonsäureethylester enthält.

b) Verfahren zur Herstellung der 2,2-Dimethyl-3-formylhydrazon-cyclopropane der Formel II

Beispiel 3

4,8 g (0,02 Mol) p-Tolylsulfonyl-acetaldehyddimethylhydrazon, 16 g (0,08 Mol) 2-Methylpropenyl-1,1-dicarbonsäureethylester wurden unter Stickstoffatmosphäre mit 120 ml abs. DMF und 4 ml abs. HMPT versetzt. Es wurden 4,08 g NaOEt (0,06 Mol) hinzugegeben und das Reaktionsgemisch 3 d bei 90°C unter Stickstoff gerührt.

Es wurde abgekühlt und auf 200 ml Eiswasser gegossen, sechsmal mit Ether extrahiert und die Etherextrakte viermal mit $H_2O$ gewaschen. Die Waschphasen

wurden ebenfalls zweimal mit Ether extrahiert. Die organischen Phasen wurden vereinigt und über $Na_2SO_4$ getrocknet. Es wurde abfiltriert und der Ether abdestilliert. Das Rohprodukt wurde auf einer Kieselgelsäule mit PE 30/50 / Ether 5:1 chromatographiert. Ausbeute an 2,2-Dimethyl-3-formyl-dimethylhydrazon-cyclopropan-1,1-diethylcarbonsäureester: 4,15 g ≙ 73 % gelbes Öl.

1HNMR: 1,1-1,5 ppm 12 Methylprotonen; 2,65 ppm D. 3-Ring-Methinproton, 2,85 ppm S. 6 Methylprotonen; 4,25 ppm Q. 4 Methylenprotonen, 6,5 ppm D. 1 Methinproton.

c) Verfahren zur Herstellung der Hydrazone der Formel III

Beispiel 4

Zu 1,2 g Dinitrophenylhydrazin gab man 6 ml konz. $H_2SO_4$, anschließend unter Rühren 9 ml $H_2O$. Der warmen Lösung setzte man 30 ml EtOH hinzu. Dieses Gemisch versetzte man mit einer Lösung von 0,65 g Phenylsulfonyl-acetaldehyddiethylacetal in 3 ml EtOH. Anschließend wurde 2 h unter Reflux gekocht, abgekühlt und Phenylsulfonyl-acetaldehyd-2,4-dinitrophenylhydrazon abfiltriert.
Ausbeute: 0,83 g ≙ 91,2 %.

1HNMR: $d_6$DMSO: D. bei 4,4 ppm 2 Methylenprotonen 7,5-8,0 ppm 10 Protonen Arom. + Methinproton + Aminproton.

Beispiel 5

1,17 g (5 mMol) Tosylacetaldehydhydrat wurden mit 150 ml $CH_2Cl_2$, 20 Tropfen konz. HCl und 2 g N,N-Dimethylhydrazin versetzt. Anschließend wurde 15 h am Wasserabscheider unter Rückfluß gekocht, 10 Tropfen HCl hinzugegeben und noch einmal 5 h unter Rückfluß gekocht. $CH_2Cl_2$ wurde abdestilliert. Der Rückstand wurde mit Ether versetzt und fünfmal mit $H_2O$ gewaschen, mit $MgSO_4$ getrocknet, filtriert und eingeengt und aus Ether/PE 60/90 auskristallisiert.

Ausbeute an Tosylacetaldehyd-dimethylhydrazon:
2 g ≙ 100 %.
Schmelzpunkt: 75°C.

1HNMR: S. 2,46 ppm 3 Methylprotonen; S. 2,7 ppm 6 Methylprotonen; D 3,9 ppm 2 Methylenprotonen; T. 6,25 ppm 1 Methinproton; 0,725 ppm und 7,75 pp 4 Proton.
D. 7,25 ppm 2 arom. Ringprotonen
D. 7,75 ppm 2 arom. Ringprotonen

Beispiel 6

2,00 g (0,01 Mol) Tosylethanol wurden mit 30 ml $CH_2Cl_2$ versetzt und 0,34 g (0,001 Mol) $Bu_4NHSO_4$ hinzugegeben. Dieses Gemisch wurde auf -5°C gekühlt und eine Lösung von 1,28 g (0,066 Mol) $K_2CrO_4$ in 12 ml 30 %iger $H_2SO_4$ innerhalb ca. 40 Min. zugetropft und 15 Min. bei -5°C gerührt. Anschließend wurde noch 3 Tage bei Raumtemperatur gerührt. Danach

wurde mit 10 ml 10 %iger $FeSO_4$ Lösung versetzt und fünfmal mit Ether/THF 1:1 extrahiert. Die organischen Phasen wurden vereinigt und zweimal mit $H_2O$ gewaschen und die organischen Lösungsmittel weitgehend abdestilliert.

Der Rückstand wurde mit 60 ml $CH_2Cl_2$, 5 ml $H_2O$, 10 Tropfen konz. HCl und 2 g N,N-Dimethylhydrazin versetzt. Anschließend wurde 14 Stunden am $H_2O$-Abscheider unter Rückfluß gekocht. Die Lösung wurde abgekühlt, dreimal mit 20 ml $H_2O$ gewaschen, über $Na_2SO_4$ getrocknet, filtriert und $CH_2Cl_2$ abdestilliert. Der Rückstand (2,05 g) enthält ca. 30 % Tosylacetaldehyd-dimethylhydrazon.

Beispiel 7

1,4 ml Diisopropylamin (0,01 Mol) wurden in 30 ml abs. THF unter Stickstoff gelöst und bei 0°C 6,4 ml einer 1,6 molaren Lösung von BuLi in n-Hexan zugespritzt. Anschließend wurde 15 Min. bei 0°C gerührt. 0,86 g (0,01 Mol) Acetaldehyddimethylhydrazon wurden nach 5 Min. zugespritzt und anschließend 15 Min. bei 0°C gerührt. Es wurde auf -78°C gekühlt und 1,91 g (0,01 Mol) p-Toluylsulfonylchlorid in 3 ml THF innerhalb ca. 7 Min. zugespritzt. Man ließ in ca. 15 h auf Raumtemperatur erwärmen und rührte noch 3 h bei Raumtemperatur, versetzte mit 50 ml $H_2O$ und extrahierte fünfmal mit Ether. Die Etherextrakte wurden dreimal mit $H_2O$ gewaschen, über $MgSO_4$ getrocknet

und destilliert. Man erhielt 1,19 g Produkt, das ca. 5 % Tosylacetaldehyddimethylhydrazon enthielt.

Analog zu den in Beispielen 4, 5, 6 und 7 beschriebenen Verfahren lassen sich die folgenden Verbindungen herstellen:

**Beispiel 8** Struktur physik.Daten

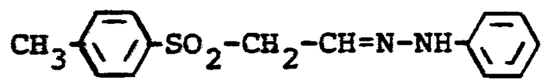

1HNMR; S. 2,4 ppm 3 Methylprotonen
D. 4,0 ppm 2 Methylenprotonen
m: 6,55-8,2 ppm 9 arom. Ringprotonen
1 Methinproton
1 Aminproton

**Beispiel 9**

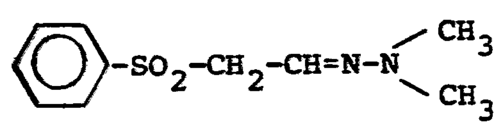

1HNMR: S. 2,7 ppm 6 Methylprotonen
D. 3,9 ppm 2 Methylenprotonen
T. 6,25 ppm 1 Methinproton
M. 7,4-8,0 ppm 5 arom. Ringprotonen

Beispiel 10

In einem mit Rührer und Stickstoffanschluß ausgestatteten 100 ml Dreihalskolben werden nach Spülen mit Stickstoff 0,48 g (mMol) 2,2-Dimethyl-3-formyl-cyclopropan-1,1-dicarbonsäureethylester in 40 ml trockenem Methylenchlorid vorgelegt. Man kühlt auf -10°C und gibt 0,594 g (3mMol) Bromtrichlormethan mittels Injektionsspritze hinzu. Danach werden 1,2 g (7,3mMol) Hexamethyltriaminphosphin in 40 ml trockenem Methylenchlorid gelöst innerhalb 30 Minuten hinzugegeben. Man läßt noch 3 h bei -10°C rühren und anschließend langsam auf Raumtemperatur erwärmen. Man versetzt die Reaktionslösung mit 100 ml n-Hexan, wäscht zweimal mit Wasser, einmal mit 0,1 n HCl und noch einmal mit Wasser, trocknet mit Magnesiumsulfat, filtriert und engt die organische Phase am Rotationsverdampfer ein. Nach säulenchromatographischer Reinigung mittels Kieselsäule und PE (30 / 50)-Ether-Gradient erhält man 0,55 g (89 % der Theorie) 3-(2,2-Dichlorvinyl)-2,2-dimethyl-cyclopropan-1,1-dicarbonsäure-diethylester

H NMR ($CDCl_3$): 1,27 ppm (T, 6H Methylprotonen),
1,27 ppm (S, 6H Methylprotonen)
2,57 ppm (D, 1H Cyclopropanringproton),
4,18 ppm (M, 4H Methylenprotonen),
5,85 ppm (D, 1H Vinylproton).

**Patentansprüche:**

1. 2,2-Dimethyl-3-formylcyclopropane der Formel I

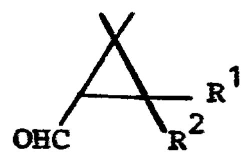

I

in welcher

$R^1$ und $R^2$ für CN, $COC_{1-4}$-Alkyl, CO Halogen, $COOC_{1-4}$-Alkyl stehen.

2. Verfahren zur Herstellung der 2,2-Dimethyl-3-formyl-cyclopropane der Formel I

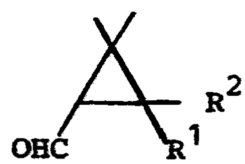

I

in welcher

$R^1$ und $R^2$ für CN, $COC_{1-4}$-Alkyl, CO Halogen, $COOC_{1-4}$-Alkyl stehen,

dadurch gekennzeichnet, daß man 2,2-Dimethyl-3-formylhydrazon-cyclopropane der Formel II

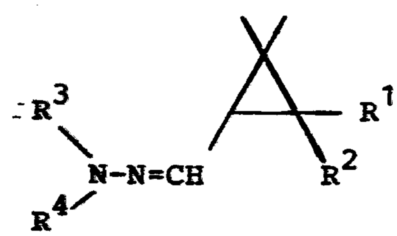

II

in welcher

$R^1$ und $R^2$ die oben angegebene Bedeutung haben und

$R^3$ und $R^4$ für Wasserstoff, $C_{1-4}$-Alkyl oder gegebenenfalls substituiertes Phenyl stehen oder gemeinsam eine Alkylenbrücke bilden, die gegebenenfalls durch Heteroatome unterbrochen und gegebenenfalls substituiert ist,

in an sich bekannter Weise spaltet.

3. 2,2-Dimethyl-3-formylhydrazon-cyclopropane der Formel II

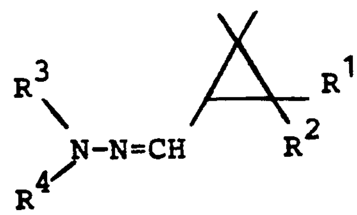

II

in welcher

$R^1$ und $R^2$ die oben angegebene Bedeutung haben und

$R^3$ und $R^4$ für Wasserstoff, $C_{1-4}$-Alkyl oder gegebenenfalls substituiertes Phenyl stehen oder gemeinsam eine Alkylenbrücke bilden, die gegebenenfalls durch Heteroatome unterbrochen und gegebenenfalls substituiert ist.

4. Verfahren zur Herstellung der 2,2-Dimethyl-3-formylhydrazon-cyclopropane der Formel II

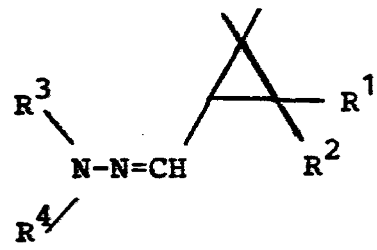

II

in welcher

$R^1$ und $R^2$ die oben angegebene Bedeutung haben und

$R^3$ und $R^4$ für Wasserstoff, $C_{1-4}$-Alkyl oder gegebenenfalls substituiertes Phenyl stehen oder gemeinsam eine Alkylenbrücke bilden, die gegebenenfalls durch Heteroatome unterbrochen und gegebenenfalls substituiert ist,

dadurch gekennzeichnet, daß man Hydrazone der Formel III

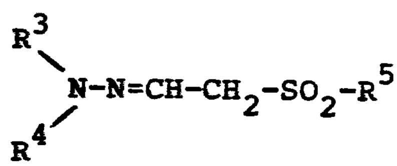

III

in welcher

$R^3$ und $R^4$ die oben angegebene Bedeutung haben und

$R^5$ für gegebenenfalls substituiertes Aryl steht,

mit Alkylenen der Formel IV

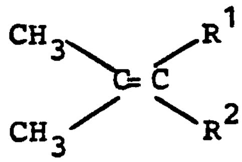

IV

in welcher

$R^1$ und $R^2$ die oben angegebene Bedeutung haben,

in Gegenwart von Basen umsetzt.

5. Hydrazone der Formel III

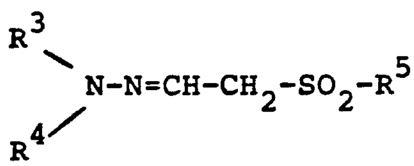

III

in welcher

$R^3$ und $R^4$ die oben angegebene Bedeutung haben und

$R^5$ für gegebenenfalls substituiertes Aryl steht.

6. Verfahren zur Herstellung der Hydrazone der Formel III

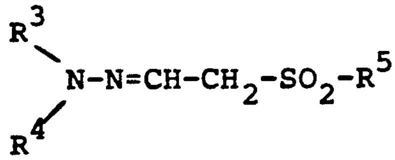

III

in welcher

$R^3$ und $R^4$ die oben angegebene Bedeutung haben und

$R^5$ für gegebenenfalls substituiertes Aryl steht,

dadurch gekennzeichnet, daß man

a) Sulfonylacetaldehydhydrate der Formel V

$$R^5-SO_2-CH_2-CHO \cdot 2H_2O \qquad V$$

in welcher

$R^5$ die oben angegebene Bedeutung hat,

mit Hydrazinen der Formel VI

$$H_2N-NR^3R^4 \qquad VI$$

in welcher

$R^3$ und $R^4$ die oben angegebene Bedeutung haben,

umsetzt oder

b) Sulfonylacetaldehydacetale der Formel VII

$$R^5-SO_2-CH_2-CH(OR^6)_2 \qquad VII$$

in welcher

$R^5$ die oben angegebene Bedeutung hat und

$R^6$ für $C_{1-4}$-Alkyl, insbesondere Methyl oder Ethyl oder beide Reste $R^6$ für eine $C_{2-3}$-Alkylenbrücke stehen,

mit Hydrazinen der Formel VI (oben) in Gegenwart saurer Katalysatoren umsetzt oder

c) Sulfonylethanol der Formel VIII

$$R^5SO_2CH_2CH_2\text{-}OH \qquad VIII$$

in welcher

$R^5$ die oben angegebene Bedeutung hat,

zunächst in einer ersten Stufe zum Aldehyd oxidiert und anschließend in einer zweiten Stufe den Aldehyd mit Hydrazinen der Formel VI (oben) umsetzt, oder

d) Sulfonylchloride der Formel IX

$$R^5SO_2Cl \qquad IX$$

in welcher

$R^5$ die oben angegebene Bedeutung hat,

mit Acetaldehydhydrazonen der Formel X

$$CH_3-CH=N-NR^3R^4 \qquad X$$

in welcher

$R^3$ und $R^4$ die oben angegebene Bedeutung haben,

in Gegenwart von organischen Lithium-Basen, z.B. Lithiumdiisopropylamid oder Butyl-lithium umsetzt.

Europäisches Patentamt

# EUROPÄISCHER RECHERCHENBERICHT

0163996

Nummer der Anmeldung

EP 85 10 5967

| EINSCHLÄGIGE DOKUMENTE | | | |
| --- | --- | --- | --- |
| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
| X | CANADIAN JOURNAL OF CHEMISTRY, Band 61, Nr. 2, Februar 1983, Seiten 217-223, National Research Council of Canada; H. ABDALLAH et al.: "Synthèse de cyclopropanes électrophiles à fonction aldèhyde libre ou masquée"<br>--- | 1 | C 07 C 69/757<br>C 07 C 109/12<br>C 07 C 147/06 |
| X | CHEMICAL ABSTRACTS, Band 95, Nr. 19, 9. November 1981, Seite 694, Nr. 168703s, Columbus, Ohio, US; M.A. VASIL'EVA et al.: "Reaction of phenoxyethenyl organyl sulfones with amines" & ZH. ORG. KHIM. 1981, 17(7), 1405-8<br>--- | 5 | |
| X | CHEMISCHE BERICHTE, 99 Jahrg., Nr. 5, 1966, Seiten 1580-1588; H.-W. WANZLICK et al.: "Chemie nucleophiler Carbene, XI'"<br>----- | 5,6 | RECHERCHIERTE SACHGEBIETE (Int. Cl.4)<br>C 07 C 69/00<br>C 07 C 109/00<br>C 07 C 147/00 |

Der vorliegende Recherchenbericht wurde für alle Patentansprüche erstellt.

| Recherchenort | Abschlußdatum der Recherche | Prüfer |
| --- | --- | --- |
| DEN HAAG | 08-08-1985 | WRIGHT M.W. |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82